# EUROPEAN PATENT APPLICATION

(11) **EP 2 671 560 A1**
(43) Date of publication of application: **11.12.2013**
(21) Application number: 13164350.4
(22) Date of filing: 18.04.2013
(51) Int. Cl.: A61H 9/00

(54) **Compression garment including bladder having reduced inflatable volume**

(30) Priority: 06.06.2012 US 201213489910
(71) Applicant: Covidien LP, Mansfield, MA 02048 (US)
(72) Inventor: Malhi, Arnaz, Watertown, MA Massachusetts 02472 (US); Rego, Richard, Mansfield, MA Massachusetts 02048 (US); Deshpande, Manish, Canton, MA Massachusetts 02021 (US); Borden, David, Northborough, MA Massachusetts 01532 (US); Largesse, Judy, Hopkinton, MA Massachusetts 01748 (US)
(74) Representative: Gray, James

(57) **Abstract**

A compression garment including a bladder having a reduced inflatable volume. Connector elements (25) positioned inside an inflatable chamber of the bladder limit expansion of the bladder for limiting a fluid volume required for fully inflating or expanding the bladder. A controller inflates and deflates the inflatable chamber for imparting compression therapy on a body part on which the garment is worn. The reduced inflation volume of the inflatable chamber reduces the demand on a pump of the controller.

## Description

### FIELD OF THE INVENTION

The present invention is directed generally to a compression garment for applying compression therapy to a body part of a wearer, and more particularly to a compression garment including a bladder having a reduced inflatable volume.

### BACKGROUND OF THE INVENTION

Among concerns for generally immobile persons are medical conditions that form blood clots, such as deep vein thrombosis (DVT) and peripheral edema. Such patients and persons include those undergoing surgery, anesthesia, and extended periods of bed rest. These blood clotting conditions generally occur in the deep veins of the lower extremities and/or pelvis. Veins such as the iliac, femoral, popliteal, and tibial veins return deoxygenated blood to the heart. When blood circulation in these veins is retarded due to illness, injury, or inactivity, there is a tendency for blood to accumulate or pool. A static pool of blood may lead to a blood clot, which can interfere with cardiovascular circulation. More seriously, the blood clot can break loose and migrate. A pulmonary embolus, which may be life threatening, can form if the blood clot blocks a pulmonary artery.

Conventional vascular compression systems include a compression garment fluidly connected to a controller that cyclically inflates the compression garment about an extremity of a patient, such as around a leg. The cyclical inflation of the compression garment enhances blood circulation and decreases the likelihood of DVT. A system of conduits connects the compression garment to the controller. Newer vascular compression garments have portable controllers that are much smaller and mountable on the compression garments allowing patients to move about freely without first removing the compression garment or disconnecting the compression garment from its controller. These newer compression garments may be worn when a patient is stationary or ambulatory and are believed to enhance patient compliance due to convenience of use. It is desirable for the portable controllers to be as small as possible to enhance the portability of the controllers and mountability of the controllers on the garment or elsewhere on the wearer.

However, smaller portable controllers require lighter, smaller components to inflate the compression garment. Smaller components, such as a pump or generator to provide the fluid flow, may not provide enough pressure or volume to provide a clinically effective pressure in the garment. Therefore, a need exists to reduce the requirements of the bladders within the compression garment.

### SUMMARY OF THE INVENTION

In one aspect, the present invention is directed to a compression garment for applying compression therapy to a selected body part of a patient, the garment generally comprising a bladder adapted for inflating to apply compression to the selected body part. The bladder comprises an inner fluid-impermeable bladder layer having an interior face and an exterior face opposite said interior face, and an outer fluid-impermeable bladder layer having an interior face and an exterior face opposite said interior face. The outer bladder layer is joined to said inner bladder layer along a chamber boundary so that the respective interior faces are in opposed relation and at least partially define a fluid-tight chamber that is selectively expandable by introducing fluid into the chamber. A connector element having an inner portion joined to the interior face of the inner bladder layer and an outer portion joined to the interior face of the outer bladder layer limits a corresponding distance between the interior face of the inner bladder layer and the interior face of the outer bladder layer thereby limiting a fluid volume required to fully expand the chamber. A fastener may be operatively connected to the bladder for securing the garment in position on the selected body part of the patient.

In another aspect, the present invention is directed to a compression garment for applying compression therapy to a selected portion of a calf of a leg of a wearer, the garment generally comprising an inner cover layer sized and shaped for at least partially covering the selected portion of the calf of the wearer and an outer cover layer joined to the inner cover layer together with the inner cover layer forming an expandable interior cavity. A fastener may be attached to at least one of the inner and outer cover layers for fastening the inner and outer cover layers in position on the selected portion of the calf of the wearer. A bladder positioned in the expandable interior cavity between the inner and outer cover layers generally comprises an inner bladder layer formed from gas impermeable sheet material having an interior face and an exterior face opposite said interior face, the inner bladder layer having openings therein and an outer bladder layer formed from gas impermeable sheet material having an interior face and an exterior face opposite said interior face. The outer bladder layer has openings therein and is joined to the inner bladder layer along a chamber boundary so that the respective interior faces are in opposed relation and define an inflatable gas-tight chamber that is selectively inflatable by introducing gas into the chamber. A plurality of connector elements joining the inner and outer bladder layers inside the chamber boundary limits a maximum volume to which the chamber is inflatable.

In yet another aspect, the present invention is directed to a compression garment for applying compression therapy to a selected portion of a calf of a leg of a wearer, the garment generally comprising an inner cover layer sized and shaped for at least partially covering the selected portion of the calf of the wearer and an outer cover layer joined to the inner cover layer together with the inner cover layer forming an expandable interior cavity. A fastener may be attached to at least one of the inner and outer cover layers can fasten the inner and outer cover layers in position on the selected portion of the calf of the wearer. A bladder positioned in the expandable interior cavity between the inner and outer cover layers comprises an inner bladder layer formed from gas impermeable sheet material having an interior face and an exterior face opposite said interior face, and an outer bladder layer formed from gas impermeable sheet material having an interior face and an exterior face opposite said interior face. The outer bladder layer is joined to the inner bladder layer along a chamber boundary so that the respective interior faces are in opposed relation and define an inflatable gas-tight chamber that is selectively inflatable by introducing gas into the chamber. A plurality of connector elements joining the inner and outer bladder layers inside the chamber boundary limit a maximum volume to which the chamber is inflatable. The connector elements are fluid impermeable and tubular in shape and communicate with the openings whereby the openings and the tubular connector define a passage through the bladder.

Other features will be in part apparent and in part pointed out hereinafter.

### BRIEF DESCRIPTION OF THE DRAWINGS

Fig. 1 is a schematic outer elevation of a compression garment of the present invention shown in an open, unwrapped configuration, an outer cover layer and intermediate layers of the sleeve being partially removed to show underlying layers;
Fig. 2 is a schematic inner elevation of the compression garment;
Fig. 3 is an schematic exploded perspective of the compression garment;
Fig. 4 is a schematic outer elevation of a bladder assembly of the compression garment;
Fig. 5 is a schematic section of the bladder assembly taken in the plane of line 5--5 in Fig. 4 illustrating the bladder assembly in a fully inflated configuration;
Fig. 6 is a schematic perspective of the section shown in Fig. 5;
Fig. 7 is an enlarged view of a portion of the section shown in Fig. 5;
Fig. 8 is a schematic outer elevation of another embodiment of a bladder assembly of the present invention;
Fig. 9 is a schematic exploded perspective of the bladder assembly of Fig. 8;
Fig. 10 is a schematic section of the bladder assembly taken in the plane of line 10--10 in Fig. 8 illustrating the bladder assembly in a fully inflated configuration;
Fig. 11 is a schematic perspective of the section shown in Fig. 10;
Fig. 12 is an enlarged view of a portion of the section shown in Fig. 11;
Fig. 13 is a schematic outer elevation of another embodiment of a bladder assembly of the present invention; and
Fig. 14 is a schematic section of the bladder assembly of Fig. 13 taken in the plane of line 14--14 in Fig. 14 illustrating the bladder assembly in a fully inflated configuration.

Corresponding reference characters indicate corresponding parts throughout the drawings.

### DETAILED DESCRIPTION OF THE DRAWINGS

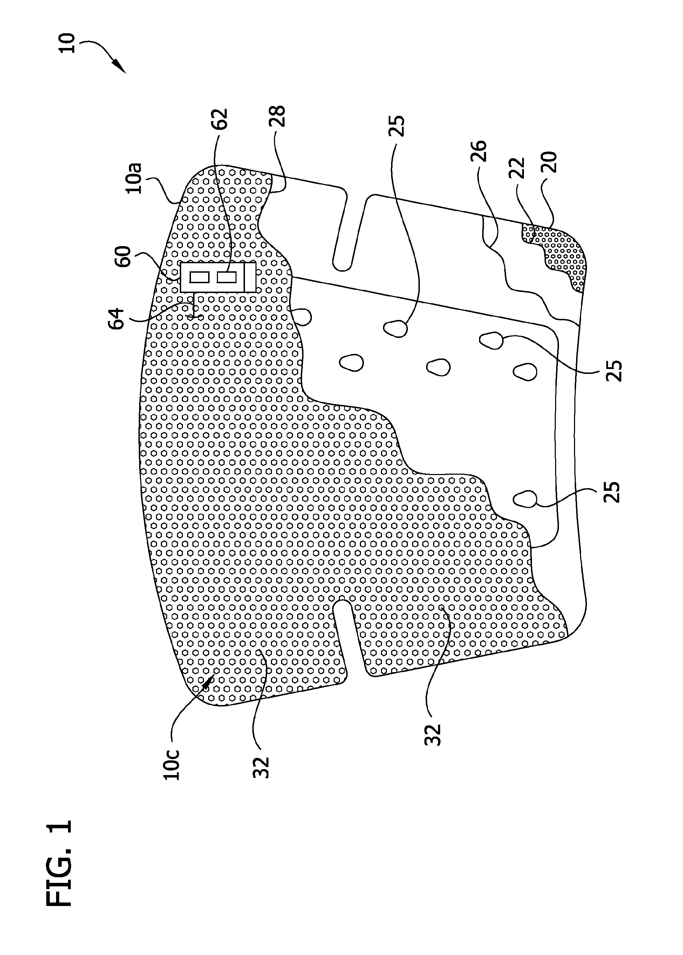

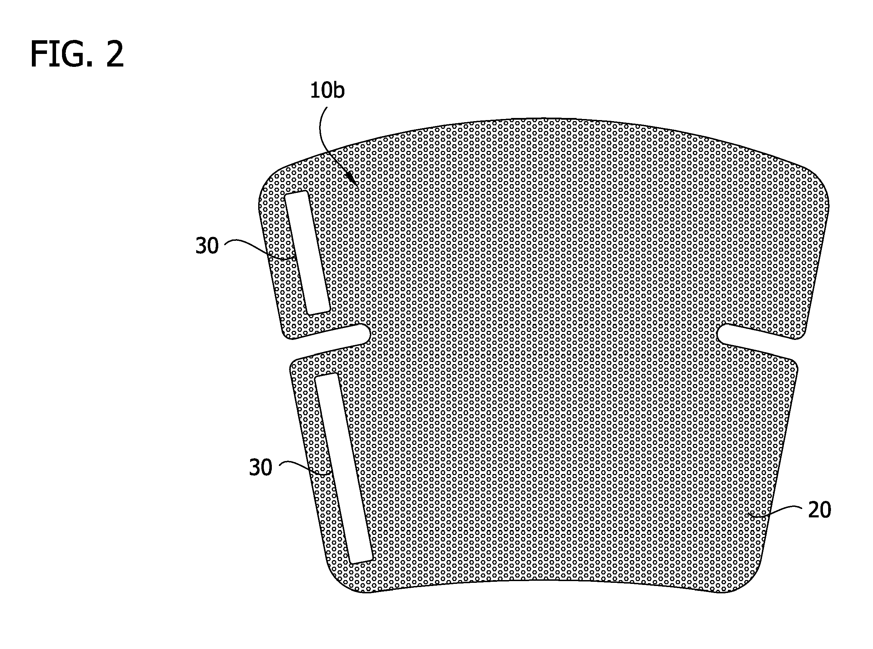

Referring now to the drawings and in particular to Figs. 1 and 2, one embodiment of a compression garment of the present invention is generally indicated by the reference number 10. The compression garment 10 is used to apply compression therapy to a selected body part of a person (e.g., a limb such as a leg or arm). The illustrated compression garment 10 is configured to be applied to a leg and has a "knee length" size, meaning the compression garment extends generally from the ankle to below the knee. The compression garment 10 is sized for applying compression therapy to a selected portion of a calf of a leg of a patient. Other sizes and shapes of garments (e.g., "thigh length," extending generally from the ankle to the thigh) and garments configured for use on other body parts may be used without departing from the scope of the present invention.

As will become apparent, the compression garment 10 is configured for limiting a fluid volume required to fully inflate an inflatable chamber of the compression garment, or required to achieve a predetermined desired end-of-cycle pressure in the inflatable chamber. In other words, the compression garment 10 is configured for minimizing a maximum volume to which the chamber is inflatable. Such a configuration reduces demand on a pump used to inflate the chamber while permitting the garment to achieve the desired gas pressure in the chamber at an end of the inflation cycle to provide sufficient DVT therapy. Accordingly, the pump may be reduced in size and/or weight, which enhances the portability of the controller and the mountability of the controller on the compression garment. Features of the present invention provide these and other advantages without reducing the efficacy of the compression therapy the compression garment 10 imparts on a body part.

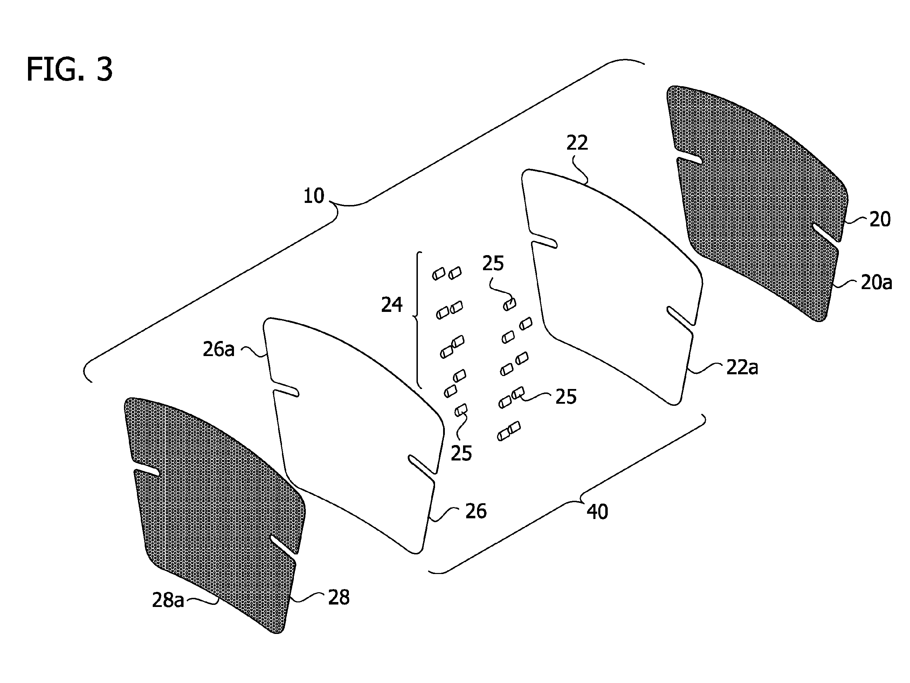

As shown in Figs. 1 and 3, the compression garment 10 has a layered construction comprising five layers. More specifically, the compression garment 10 includes an inner cover layer 20, an inner bladder layer 22, a layer 24 including a plurality of connector elements 25, an outer bladder layer 26, and an outer cover layer 28. As used herein, the terms "inner" and "outer" refer to the position of the layers when wrapped around the body part. For example, when the compression garment 10 is wrapped on the limb, the inner cover layer 20 is closer to the limb and the outer cover layer 22 is farther from the limb. The cover layers 20, 28 are sized and shaped for at least partially covering the selected portion of the body part (e.g., a selected portion of the calf) to which compression therapy is to be applied.

The compression garment 10 is configured to be wrapped around a body part and includes at least one fastener for fastening the garment in position on the body part. For example, the garment may be secured in position using hook-and-loop fasteners. As shown in Fig. 2, sections of hook fabric 30 are provided on the inner cover layer 20 and spaced apart longitudinally along a side margin of the compression garment 10, generally indicated by the reference number 10b. The garment 10 is wrapped around the body part and secured in position by engaging the sections of hook fabric 30 with loop fabric 32 on the outer cover layer 28 at an opposite side margin of the compression garment, generally indicated by the reference number 10c (Fig. 1). Persons having ordinary skill in the art will appreciate that other types of fasteners may be used to secure the garment 10 in position without departing from the scope of the present invention.

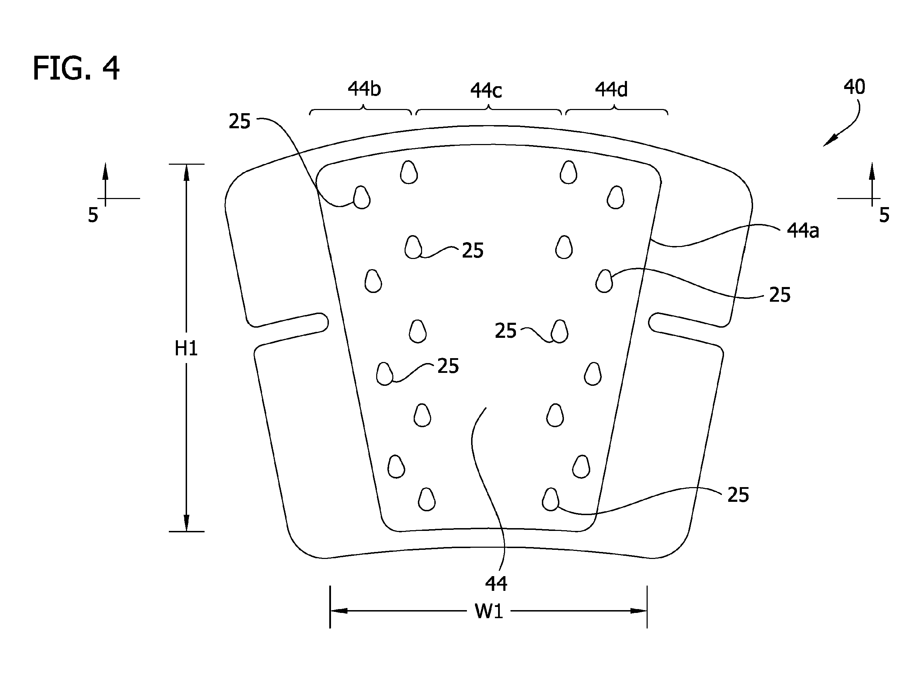

The inner and outer bladder layers 22, 26 sandwich the layer 24 of connector elements 25 to form a bladder assembly 40 (Figs. 3 and 4), and the inner and outer cover layers 20, 28 sandwich the bladder assembly, positioning the layers in a stacked configuration. As shown in Fig. 3, the cover layers 20, 28 and the bladder layers 22, 26 have respective perimeters 20a, 28a, 22a, 26a. As illustrated, all of the layers 20, 22, 26, 28 have generally the same size and shape. When the compression garment 10 is constructed, the perimeters 20a, 28a, 22a, 26a are generally in register with each other. The perimeters of the cover layers 20a, 28a define a perimeter of the compression garment 10a (Fig. 1). More or fewer layers may be used without departing from the scope of the present invention. Moreover, layers having differing sizes and shapes may be used without departing from the scope of the present invention. For example, the cover layers and bladder layers may have other perimeter sizes and shapes and may not be in register with each other.

The inner cover layer 20 is joined to the outer cover layer 28 to form an expandable interior cavity inside which the bladder assembly 40 is positioned. For example, the inner and outer cover layers 20, 28 may be joined at their perimeters 20a, 28a through the bladder layers 22, 26. The cover layers 20, 28 may be joined by welding, such as radiofrequency welding or heat sealing. Other types of mechanical and/or chemical processes may be used. Moreover, the cover layers 20, 28 may be joined to each other and/or to the bladder layers 22, 26 at other locations without departing from the scope of the present invention.

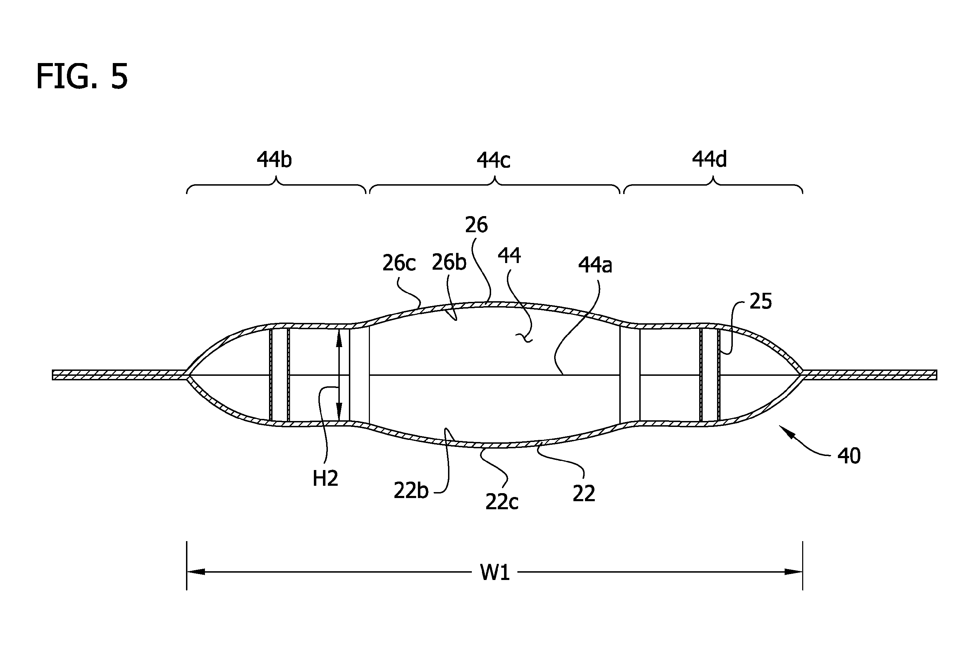

The inner and outer bladder layers 22, 26 are fluid-impermeable. As shown in Fig. 5, the bladder layers 22, 26 are joined together to form at least one fluid-tight inflatable chamber 44 that is selectively expandable by introducing fluid into the chamber. More specifically, each of the bladder layers 22, 26 includes an interior face 22b, 26b and an exterior face 22c, 26c. When the inner and outer bladder layers 22, 26 are joined together, the interior faces 22b, 26b of the bladder layers 22, 26 are positioned in opposed relation and at least partially define the inflatable chamber 44. The bladder layers 22, 26 are joined or sealed together at a chamber boundary or perimeter 44a. The bladder layers 22, 26 may be joined using similar techniques as for the cover layers 20, 28. For example, the bladder layers 22, 26 may be joined at the chamber boundary 44a by welding, such as radiofrequency welding or heat sealing. Other types of mechanical and/or chemical processes may be used. In the illustrated embodiment, as shown in Fig. 4, the bladder assembly 40 includes one inflatable chamber 44. Other numbers of inflatable chambers (e.g., two, three, four, five, or more chambers) may be used without departing from the scope of the present invention.

The connector elements 25 of layer 24 are provided for limiting a distance which the interior surfaces 22a, 26a of the bladder layers 22, 26 can move away from each other when the chamber 44 is fully inflated, or inflated to a desired predetermined end-of-inflation-cycle pressure such as for imparting sufficient DVT compression therapy on the body part. Accordingly, the connector elements 25 limit a fluid volume required to fully inflate or expand the chamber 44. In other words, the connector elements 25 minimize a maximum volume to which the chamber 44 is inflatable. As explained in further detail below, the connector elements 25 may permit certain portions of the inner and outer bladder layers 22, 26 to move a greater distance away from each other than other portions of the bladder layers.

Figure 5 illustrates a section of the bladder assembly 40 as indicated in Fig. 4 wherein the inflatable chamber 44 is in a fully inflated configuration. As shown in Fig. 5, the connector elements 25 are positioned inside the inflatable chamber 44. As shown in Figs. 4 and 5, the connector elements 25 are positioned inside or inboard from the chamber boundary 44a and are spaced from the chamber boundary. In the illustrated embodiment, 18 connector elements 25 are provided in an alternating pattern along the height of and adjacent the sides of the inflatable chamber 44. Other numbers of connector elements 25 (e.g., one, two, four, eight, ten or more connector elements) and connector elements in other arrangements may be used without departing from the scope of the present invention.

Figures 5-7 show the inner and outer bladder layers 22, 26 both bulging due to the full inflation of the chamber 44. It is understood in use, when applied on a body part and fully inflated, the bladder 40 would have a different profile than shown. For example, the outer bladder layer 26 would bulge less than shown. It is understood the garment 10 may be wrapped on the body part in such a way that outer cover layer 28 may limit bulging of the outer bladder layer 26 outward from the body part so that expansion of the inflatable chamber 44 is directed primarily toward the body part to compress the body part. Moreover, other structure such as a bladder backing layer (not shown) may be provided to promote expansion of the inflatable chamber 44 toward the body part.

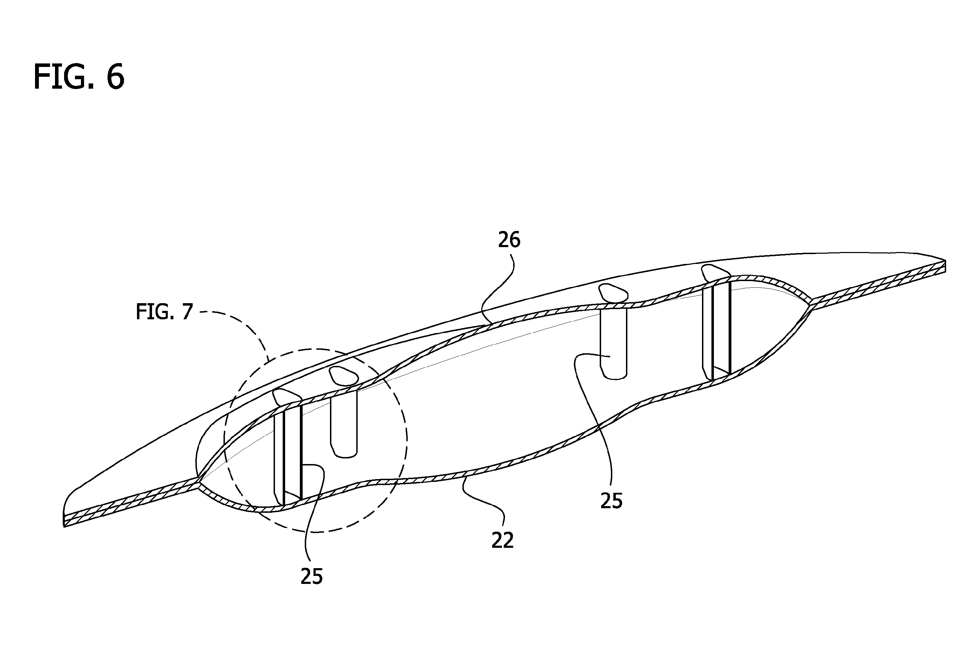

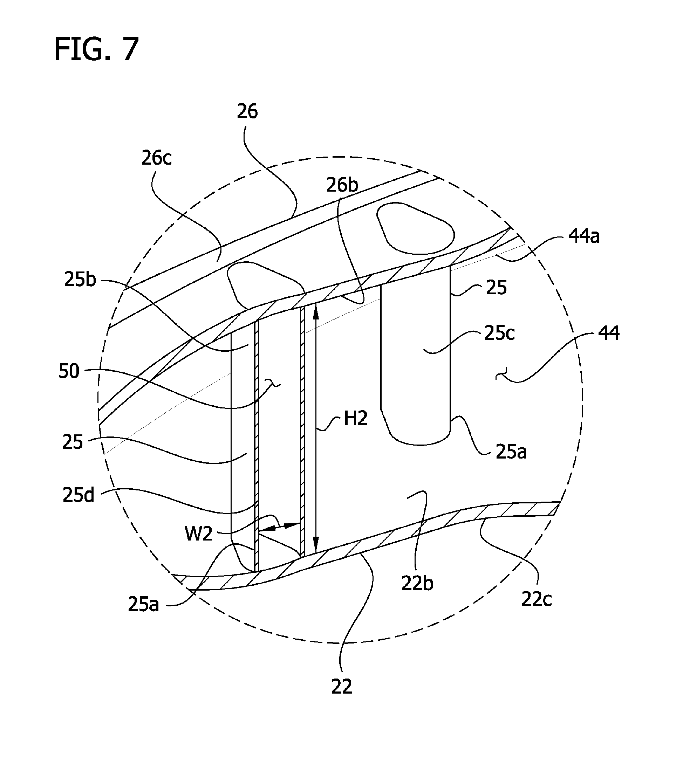

Referring to Figs. 6 and 7, the connector elements 25 have opposite ends which are connected or sealed to the inner and outer bladder layers 22, 26. More specifically, the connector elements 25 each have an inner margin 25a at an inner end connected to the interior surface 22b of the inner bladder layer 22 and an outer margin 25b at an outer end connected to the interior surface 26b of the outer bladder layer 26. The inner and outer margins 25 a, 25b connect the connector elements to the interior surfaces 22b, 26b of the inner and outer bladder layers 22, 26. In one embodiment, the connector elements 25 are formed separately from the bladder layers 22, 26 and are then connected to the bladder layers during manufacture of the bladder assembly. The connector elements 25 may be joined to the inner and outer bladder layers 22, 26 by welding, such as radiofrequency welding or heat sealing. Other types of mechanical and/or chemical processes may be used.

In the illustrated embodiment, the connector elements 25 are generally tubular columns having a cross section which is generally teardrop or egg shaped. As shown in Fig. 7, an exterior surface 25c of each tubular column 25 partially defines the inflatable chamber 44, and an interior surface 25d of each tubular column defines or surrounds a hollow space 50 inside the connector element. The hollow space 50 may be closed at the inner end of the tubular column 25 by the interior surface 22b of the inner bladder layer 22 and closed at the outer end of the tubular column by the interior surface 26b of the outer bladder layer 26. Connector elements which are not tubular or that have other cross sections may be used without departing from the scope of the present invention.

As shown in Figs. 4-7, the connector elements 25 are configured to permit the bladder layers 22, 26 to move away from each other at and adjacent the location of the connector elements 25 such that the inflatable chamber 44 can expand across substantially the entire width W1 (Figs. 4 and 5) and substantially the entire height H1 (Fig. 4) of the inflatable chamber 44 inside or inboard of the chamber perimeter 44a. The connector elements 25 are configured so when the chamber 44 is fully inflated the bladder layers 22, 26 are free from contact with each other at and adjacent the connector members 25. Thus, when the inflatable chamber 44 is fully inflated (to the desired pre-determined end-of-inflation-cycle pressure for imparting sufficient DVT compression therapy), the entire portions of the interior surfaces 22b, 26b of the inner and outer bladder layers 22, 26 which form the chamber 44 may be free from direct contact inside or inboard from the chamber boundary or periphery 44a. Accordingly, in use the chamber 44 is capable of imparting at least some level of compression to the entire portion of the body part which underlies the chamber 44. This is because the entire portion of the inner bladder layer 22 which forms part of the inflatable chamber 44 is permitted to expand at least to some degree away from the portion of the outer bladder layer 26 which forms part of the inflatable chamber 44.

In comparison, if the connector elements 25 were replaced with direct connections of the interior surfaces 22b, 26b of the bladder layers 22, 26 (e.g., if the bladder layers 22, 26 were welded directly together in interior face-to-face relationship), isolated portions of the inner bladder layer, i.e., portions of the inner bladder layer at the direct connections, would not expand away from the outer bladder layer toward the body part. Direct connections of the bladder layers 22, 26 do not permit any movement of the bladder layers away from each other at the location of the direct connections. In the present invention, because the connector elements 25 permit at least some expansion of the inflatable chamber 44 at the locations of the connector elements, the connector elements 25 provide the inflatable chamber 44 with an enhanced profile when fully inflated compared to embodiments in which the bladder layers 22, 26 are connected directly together in interior face-to-face relationship. Although it may be beneficial to not have any direct connections between the bladder layers 22, 26 inboard from the chamber perimeter 44a, it is understood one or more of such direct connections may be provided in combination with the connector elements 25 without departing from the scope of the present invention.

The connector elements 25 may be arranged in various positions in the inflatable chamber 44. In one embodiment, the connector elements 25 are positioned in the inflatable chamber 44 so they do not overlie certain critical compression areas of the body part. For example, in the illustrated embodiment, the connector elements 25 are positioned on the bladder assembly 40 so in use the connector elements 25 overlie portions of the leg other than a rear side of the calf. As shown in Figs. 4 and 5, the inflatable chamber 44 includes a left side region 44b, an intermediate region 44c, and a right side region 44d. In use, the intermediate region 44c overlies the rear side of the calf, and the left and right side regions 44b, 44d overlie sides of the calf. The connector elements 25 are provided along the height of the left and right side regions 44b, 44d and not on the intermediate region 44c. The connector elements 25 limit overall expansion of the bladder 40 and particularly limit expansion of the left and right side regions 44b, 44d. More specifically, the connector elements 25 each limit movement of the inner bladder layer 22 away from the outer bladder layer 26 to a distance about equal to the height H2 (Fig. 7) of the connector element 25. The intermediate region 44c is permitted to expand more than the side regions 44b, 44d for applications in which compression of the rear side of the calf is considered to be more critical than compression of the sides of the calf. As explained above, the connector elements 25 permit the entire portion of the inner bladder layer 22 forming the chamber 44 to expand toward the body part, but given the positioning of the connector elements the portion of the inner bladder layer at the intermediate region 44c of the chamber 44 is permitted to expand the most toward the body part. The connector elements 25 may be said to provide "localized" limitation of movement of the inner bladder layer 22 away from the outer bladder layer 26 at the locations of and immediately adjacent the connector elements 25. Other arrangements of connector elements may be used without departing from the scope of the present invention.

The height H2 of the connector elements 25 may be about 0.1, 0.3, 0.5, or more inches, or range from about 0.05 to 1.0 inches. In one embodiment, the height H2 of the connector elements is about 0.5 inches. The connector elements 25 limit movement of the inner bladder layer 22 away from the outer bladder layer 26 at the location of the connector elements a distance corresponding to the height H2. The connector elements 25 may have a width W2 of about 0.3, 0.4, 0.5, or more inches, or ranging from about 0.3 to 1.0 inches. In one embodiment, the width W2 of the connector elements 25 is about 0.5 inches. The positioning, size, and shape of the connector elements 25 depends on several variables such as desired compression force and size and shape of the inflatable chamber 44. In some embodiments, the size and shape of one or more connector elements 25 may be different than other connector elements 25. Numbers, sizes, and shapes of connector elements other than described herein may be used without departing from the scope of the present invention.

Various suitable materials may be used for forming the components of the compression garment. For example, the cover layers 20, 28 may comprise woven and non-woven cloth or fabric made of, for example, polyester. The bladder layers 22, 26 may be formed from gas impermeable sheet material such as a pliable PVC material having a thickness in a range from about 0.002 inches to about 0.020 inches. In one embodiment, the material is about 0.006 inches thick. The connector elements 25 may also comprise a pliable PVC material. The connector elements 25 may have a tubular wall thicknesses ranging from about 0.002 inches to about 0.010 inches. In one embodiment, the wall thickness is 0.060 inches. Other materials and thicknesses may be used without departing from the scope of the present invention.

Referring again to Fig. 1, a controller, generally indicated by the reference number 60, may be mounted on the compression garment 10. The controller 60 includes a pressurizer 62 (e.g., a pump) for supplying pressurized fluid to the inflatable chamber for inflating the chamber. The controller 60 is connected in fluid communication with the inflatable chamber 44 via a conduit 64 (Fig. 1). The controller 60 regulates compression therapy applied to the body part on which the garment 10 is worn by increasing and decreasing the gas pressure in the inflatable chamber 44. For example, the controller 60 may be programmed to cyclically inflate the chamber 44 to a desired predetermined end-of-cycle pressure for imparting sufficient DVT compression therapy and to deflate the inflatable chamber 44 to apply intermittent compression therapy as known in the art. Other types of compression therapy may also be programmed without departing from the scope of the present invention. As described above, the connector elements 25 limit the volume of fluid required to fully inflate or expand the inflatable chamber 44. Thus, the connector elements 25 reduce the demand on the pump 62 and permit for use of a smaller and/or lighter pump, which may make the controller 60 lighter and more easily mountable on the compression garment 10. Other types of controllers, including controllers not supported by the compression garment, may be used without departing from the scope of the present invention.

In use, the compression garment 10 is wrapped on a body part such as a leg. The controller 60 is activated to inflate and deflate the inflatable chamber 44 to impart compression therapy to the body part. The connector elements 25 limit a fluid volume required to fully expand the inflatable chamber 44 by limiting expansion of the chamber. Accordingly, sufficient compressive pressures may be achieved with introduction of less fluid into the chamber compared to if the connector elements 25 were not provided.

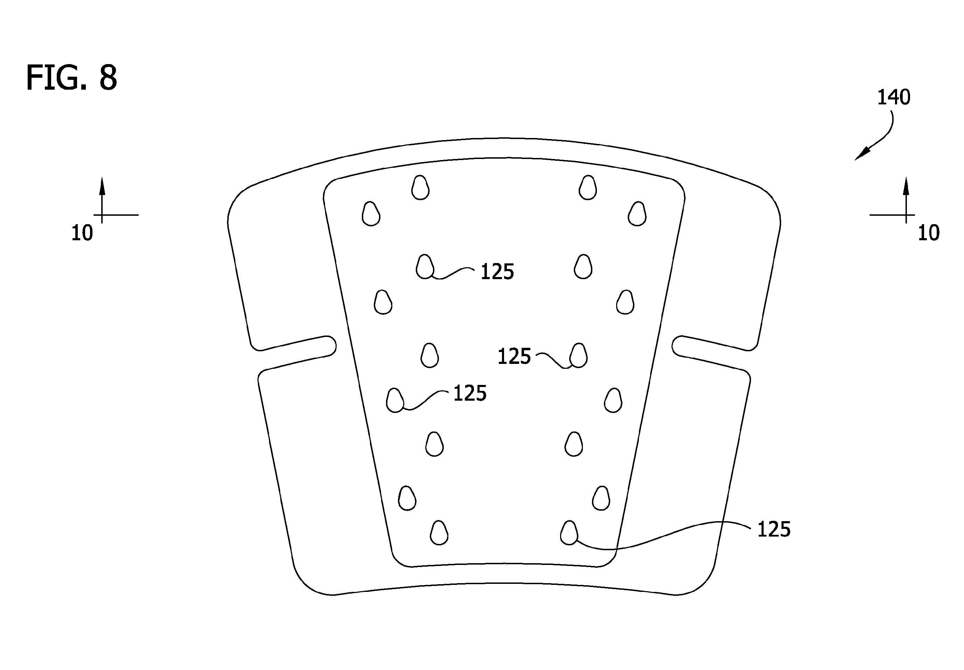

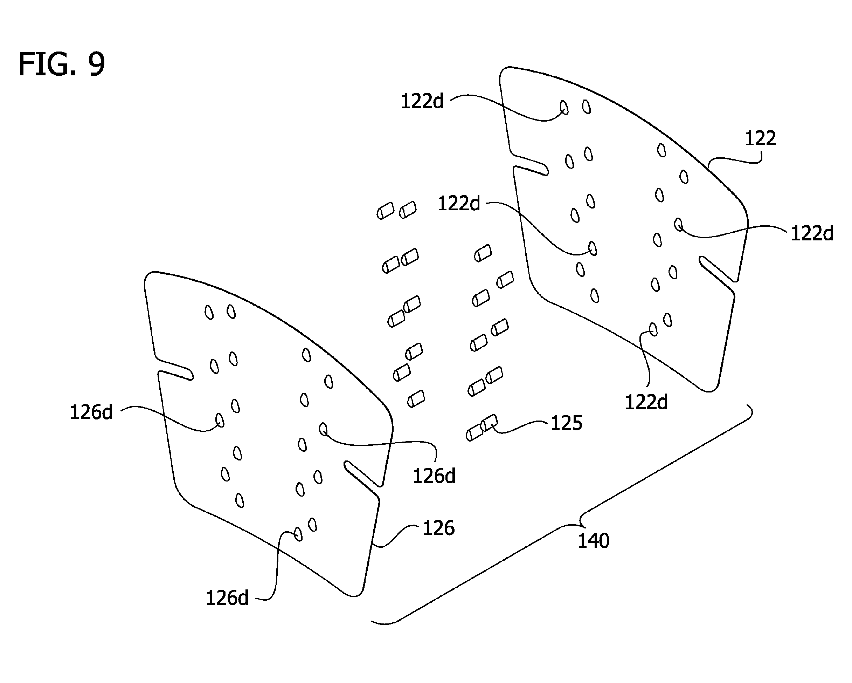

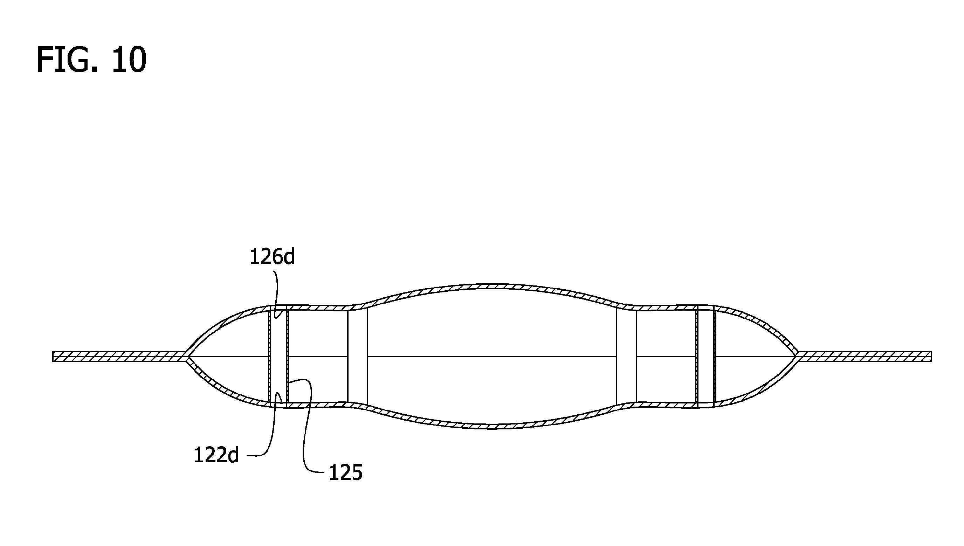

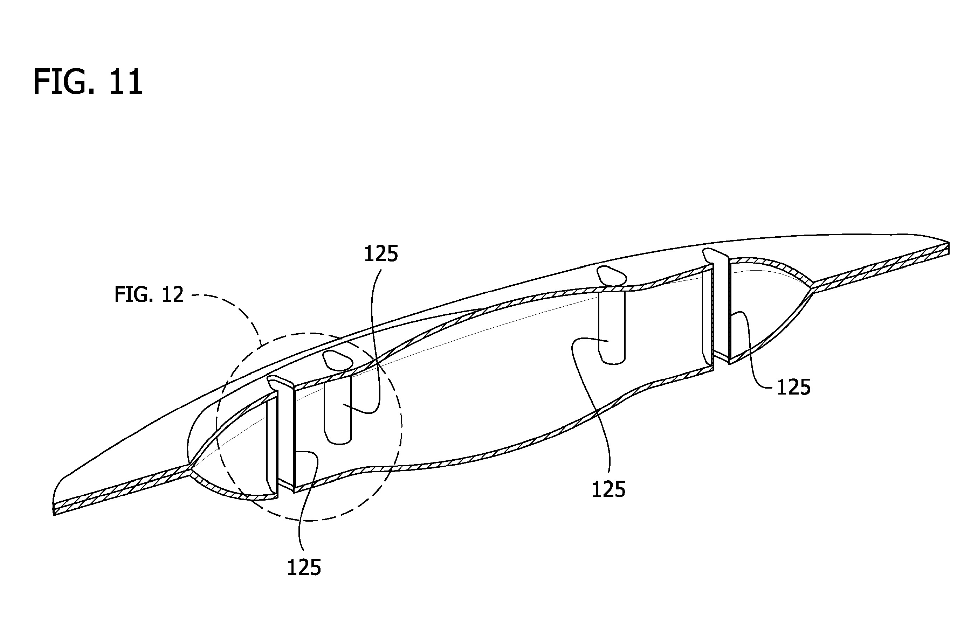

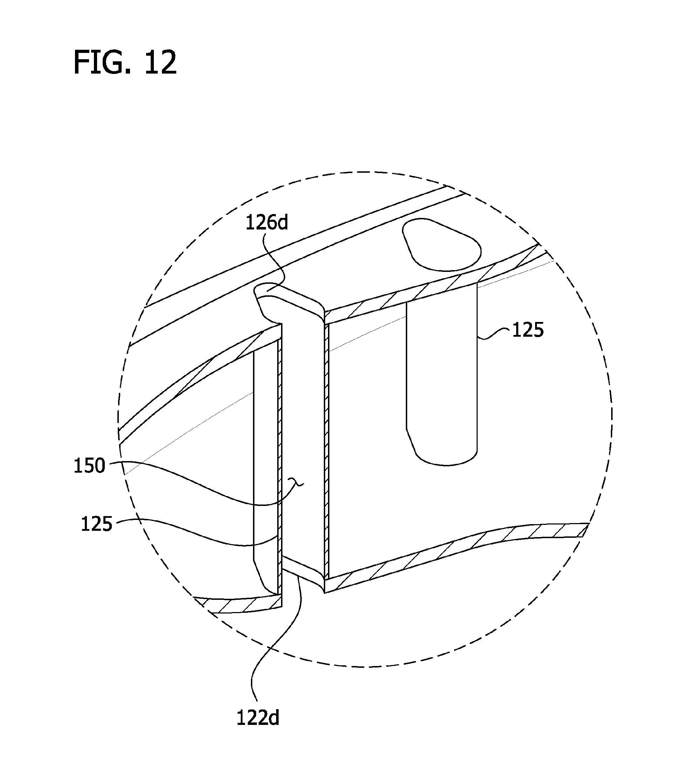

Figures 8-12 illustrate another embodiment of bladder assembly 140 of the present invention. The bladder assembly is substantially similar to the bladder assembly 40 described above, and corresponding parts are indicated by corresponding reference numbers, plus 100. As shown in Fig. 8, the bladder assembly 140 has a profile similar to the profile of the bladder assembly 40 and a similar arrangement of connector elements 125. Referring to Fig. 9, in this embodiment, the inner and outer bladder layers 122, 126 have openings or holes 122d, 126d at locations corresponding to the locations of the connector elements 125. The openings 122d, 126d are sized and shaped to generally correspond to the size and shape of the cross section of the connector members 125, which in the illustrated case is a teardrop or egg shape. As shown in Figures 11 and 12, when the bladder assembly 140 is assembled, the ends of the tubular connector elements 125 are positioned in register with the openings 122d, 126d in the bladder layers 122, 126 such that gas (other than the compression gas) is permitted to enter the hollow space 150 inside the connector elements 125. It is understood the bladder assembly 140 may be covered with cover layers similar to cover layers 20, 28 described above, and such cover layers would desirably be gas permeable such that gas, for example ambient air, can flow through the bladder assembly 140 via the hollow space 150 of each connector element 125. In other words, in this embodiment, the hollow space 150 inside the connector element 125 is not closed on either end. Such an arrangement may enhance ventilation or circulation of air to the body part on which the garment is worn and thus increase comfort for the person wearing the garment. The inner cover layer 120 can be a wicking layer capable of transporting liquid within the wicking layer from regions having more liquid to regions having less liquid could be used. The wicking layer would preferably be adjacent to the skin when the garment is worn.

In an alternative embodiment (not shown), only one of the bladder layers includes openings which permit gas to enter the hollow space in the connector element. For example, the outer bladder layer may have openings as shown in Fig. 9 but not the inner bladder layer. Thus, the outer ends of the connector elements would be open and the inner ends closed.

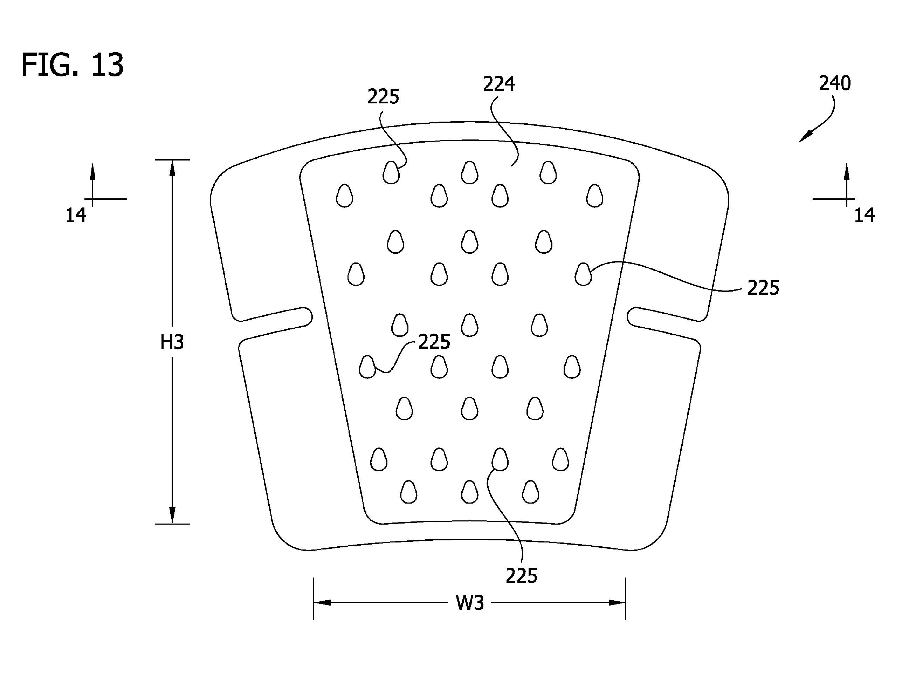

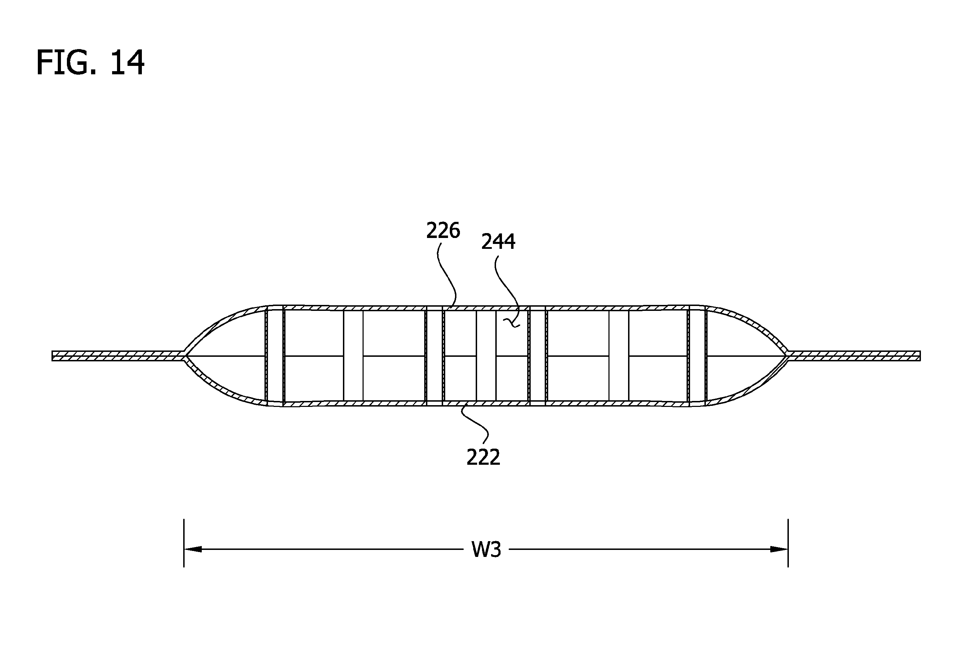

Figures 13 and 14 illustrate another embodiment of bladder assembly 240 of the present invention. The bladder assembly is substantially similar to the bladder assembly 40 described above, and corresponding parts are indicated by corresponding reference numbers, plus 200. As shown in Fig. 13, the bladder assembly 240 has a profile similar to the profile of the bladder assembly 40 but has a different arrangement of connector elements 225. In this embodiment, the connector elements 225 are arranged generally uniformly across substantially the entire height H3 and width W3 of the inflatable chamber 244. As shown in Fig. 14, such an arrangement of connector elements 225 limits expansion of the inflatable chamber 244 generally uniformly across substantially the entire height H3 and width W3 of the inflatable chamber. In other words, the connector elements 225 permit and limit movement of the portion of the inner bladder layer 222 forming the inflatable chamber 244 away from the portion of the outer bladder layer 226 forming the inflatable chamber substantially uniformly across the height H3 and width W3 of the inflatable chamber. It is understood the inner and/or outer bladder layers 222, 226 may include openings as in the embodiment shown in Figs. 9 and 12 so the connector elements 225 provide ventilation or the bladder layers may not include such openings as in the embodiment shown in Figs. 3 and 7..

While the connector elements 25, 125, 225 have been shown as tear-drop or waterdrop shaped, it is envisioned that the openings in the bladders and connector elements may be any shape, such as round, oval, square or rectangular, without departing from the scope of the claims.

When introducing elements of the present invention or the preferred embodiments(s) thereof, the articles "a," "an," "the," and "said" are intended to mean that there are one or more of the elements. The terms "comprising," "including," and "having" are intended to be inclusive and mean that there may be additional elements other than the listed elements.

In view of the above, it will be seen that the several objects of the invention are achieved and other advantageous results attained.

As various changes could be made in the above constructions, products, and methods without departing from the scope of the invention, it is intended that all matter contained in the above description and shown in the accompanying drawings shall be interpreted as illustrative and not in a limiting sense.

## Claims

1. A compression garment for applying compression therapy to a selected body part of a patient, the garment comprising:
a bladder adapted for inflating to apply compression to the selected body part , said bladder comprising:
an inner fluid-impermeable bladder layer having an interior face and an exterior face opposite said interior face;
an outer fluid-impermeable bladder layer having an interior face and an exterior face opposite said interior face, said outer bladder layer being joined to said inner bladder layer along a chamber boundary so that the respective interior faces are in opposed relation and at least partially define a fluid-tight chamber that is selectively expandable by introducing fluid into the chamber; and
a connector element having an inner portion joined to the interior face of the inner bladder layer and an outer portion joined to the interior face of the outer bladder layer for limiting a corresponding distance between the interior face of the inner bladder layer and the interior face of the outer bladder layer thereby limiting a fluid volume required to fully expand the chamber.

2. A garment as set forth in claim 1 wherein the connector element is made of a pliable material.

3. A garment as set forth in claim 1 or claim 2 wherein the connector element is tubular, the inner portion comprising an inner edge margin of the tubular element and the outer portion comprising an outer edge margin of the tubular element.

4. A garment as set forth in claims 3 wherein the connector element is fluid impermeable.

5. A garment as set forth in claim 4 wherein the inner fluid impermeable layer and outer impermeable layer having openings, the tubular connector element communicating with the openings whereby the openings and the tubular connector define a passage through the bladder.

6. A garment as set forth in claims 5 wherein the connector element has a teardrop cross section.

7. A garment as set forth in any preceding claim further comprising an inner cover layer and an outer cover layer, the inner and outer cover layers enveloping the bladder.

8. A garment as set forth in any preceding claim wherein the inner bladder layer and the outer bladder layer are free from contact with each other adjacent the connector element when the chamber is fully inflated.

9. A garment as set forth in claim 8 wherein the connector element is a first connector element and the bladder comprises a plurality of spaced connector elements including said first connector element.

10. A garment as set forth in claim 9 wherein each connector element of said plurality of connector elements has an outer margin at an outer end of the respective element and an inner margin at an inner end of the respective connector element, the outer and inner margins being connected to the interior faces of the corresponding inner and outer bladder layers.

11. A garment as set forth in claim 10 wherein the connector elements are arranged in spaced apart groups.

12. A garment as set forth in claim 11 wherein the connector elements are positioned on the bladder so in use the connector elements overlie portions of a leg of the patient other than a rear side of a calf.

13. A garment as set forth in claim 11 wherein the bladder has left and right side regions and an intermediate region between the left and right side regions, a greater number of connector elements being located in the left and right side regions that in the intermediate region.

14. A garment as set forth in claim 13 wherein the central portion is free of connector elements.

15. A compression garment as set forth in claim 9 wherein the connector elements are arranged to allow differing amounts of expansion of the bladder in different regions of the bladder.
